# EUROPÄISCHE PATENTSCHRIFT

(11) **EP 1 575 958 B1**
(45) Veröffentlichungstag und Bekanntmachung des Hinweises auf die Patenterteilung: **06.02.2008**
(21) Anmeldenummer: 03789291.6
(22) Anmeldetag: 16.12.2003
(51) Int. Cl.: C07D 487/04, A01N 43/653

(54) **FUNGIZIDE TRIAZOLOPYRIMIDINE, VERFAHREN ZU IHRER HERSTELLUNG UND IHRE VERWENDUNG ZUR BEKÄMPFUNG VON SCHADPILZEN SOWIE SIE ENTHALTENDE MITTEL**
FUNGICIDAL TRIAZOLOPYRIMIDINES, METHOD FOR THE PRODUCTION THEREOF, USE THEREOF FOR CONTROLLING HARMFUL FUNGI, AND AGENTS CONTAINING SAID FUNGICIDAL TRIAZOLOPYRIMIDINES
TRIAZOLOPYRIMIDINES FONGICIDES, PROCEDES POUR LES PRODUIRE, LEUR UTILISATION POUR LUTTER CONTRE DES CHAMPIGNONS PARASITES ET AGENTS CONTENANT CES COMPOSES

(30) Priorität: 17.12.2002 DE 10259268
(43) Veröffentlichungstag der Anmeldung: 21.09.2005
(73) Patentinhaber: BASF Aktiengesellschaft, 67056 Ludwigshafen (DE)
(72) Erfinder: MÜLLER, Bernd, 67227 Frankenthal (DE); TORMO I BLASCO, Jordi, 69514 Laudenbach (DE); GROTE, Thomas, 67157 Wachenheim (DE); BLETTNER, Carsten, 68165 Mannheim (DE); GEWEHR, Markus, 56288 Kastellaun (DE); GRAMMENOS, Wassilios, 67071 Ludwigshafen (DE); GYPSER, Andreas, 68159 Mannheim (DE); RHEINHEIMER, Joachim, 67063 Ludwigshafen (DE); SCHÄFER, Peter, 67308 Ottersheim (DE); SCHIEWECK, Frank, 67258 Hessheim (DE); SCHWÖGLER, Anja, 68165 Mannheim (DE); AMMERMANN, Eberhard, 64646 Heppenheim (DE); STRATHMANN, Siegfried, 67117 Limburgerhof (DE); SCHÖFL, Ulrich, 68782 Brühl (DE); STIERL, Reinhard, 67251 Freinsheim (DE)
(74) Vertreter: Pohl, Michael Friedrich
(86) Internationale Anmeldenummer: PCT/EP2003/014283
(87) Internationale Veröffentlichungsnummer: WO 2004/055018

(56) Entgegenhaltungen:
- EP-A- 0 071 792
- EP-A- 0 550 113
- WO-A-94/20501
- WO-A-99/41255
- WO-A-03/004465
- WO-A-03/080615

## Beschreibung

Die vorliegende Erfindung betrifft Triazolopyrimidine der Formel I, 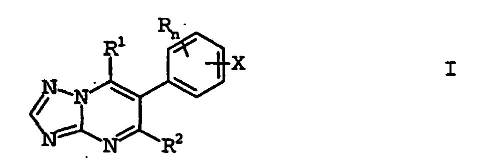 in der der Index und die Substituenten folgende Bedeutung haben:
- R¹: C₁-C₁₀-Alkyl, C₂-C₁₀-Alkenyl, C₂-C₁₀-Alkinyl, C₃-C₁₀-Cycloalkyl, C₃-C₁₀-Cycloalkenyl, Phenyl, Naphthyl, oder ein fünf- bis zehngliedriger gesättigter, partiell ungesättigter oder aromatischer Heterocyclus, der über Kohlenstoff mit dem Triazolopyrimidin verbunden ist und ein bis vier Heteroatome aus der Gruppe O, N oder S enthält,
wobei R¹ partiell oder vollständig halogeniert oder durch eine bis vier gleiche oder verschiedene Gruppen R^{a} substituiert sein kann:
R^{a} Halogen, Cyano, Nitro, Hydroxy, C₁-C₆-Alkyl, C₁-C₆-Halogenalkyl, C₁-C₆-Alkylcarbonyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₂-C₆-Alkenyl, C₂-C₆-Alkenyloxy, C₃-C₆-Alkinyloxy, C₃-C₆-Cycloalkyl, Phenyl, Naphthyl, fünf- bis zehngliedriger gesättigter, partiell ungesättigter oder aromatischer Heterocyclus, enthaltend ein bis vier Heteroatome aus der Gruppe O, N oder S, wobei diese aliphatischen, alicyclischen oder aromatischen Gruppen ihrerseits partiell oder vollständig halogeniert sein oder eine bis drei Gruppen R^{b} tragen können:
R^{b} Halogen, Cyano, Nitro, Hydroxy, Mercapto, Amino, Carboxyl, Aminocarbonyl, Aminothiocarbonyl, Alkyl, Haloalkyl, Alkenyl, Alkinyl, Alkenyloxy, Alkinyloxy, Alkoxy, Halogenalkoxy, Alkylthio, Alkylamino, Dialkylamino, Formyl, Alkylcarbonyl, Alkylsulfonyl, Alkylsulfoxyl, Alkoxycarbonyl, Alkylcarbonyloxy, Alkylaminocarbonyl, Dialkylaminocarbonyl, Alkylaminothiocarbonyl, Dialkylaminothiocarbonyl, wobei die Alkylgruppen in diesen Resten 1 bis 6 Kohlenstoffatome enthalten und die genannten Alkenyl- oder Alkinylgruppen in diesen Resten 2 bis 8 Kohlenstoffatome enthalten und die vorgenannten Gruppen teilweise oder vollständig halogenisiert sein können;
und/oder einen bis drei der folgenden Reste:
Cycloalkyl, Cycloalkoxy, Heterocyclyl, Heterocyclyloxy, wobei die cyclischen Systeme 3 bis 10 Ringglieder enthalten; Aryl, Aryloxy, Arylthio, Aryl-C₁-C₆-alkoxy, Aryl-C₁-C₆-alkyl, Hetaryl, Hetaryloxy, Hetarylthio, wobei die Arylreste vorzugsweise 6 bis 10 Ringglieder, die Hetarylreste 5 oder 6 Ringglieder enthalten, wobei die cyclischen Systeme partiell oder vollständig halogeniert oder durch Alkyl- oder Haloalkylgruppen substituiert sein können;
- R²: C₁-C₄-Alkyl, das durch Halogen, Cyano, Nitro oder C₁-C₂-Alkoxy substituiert sein kann;
- n: 0 oder eine ganze Zahl von 1 bis 4;
- R: Halogen, Cyano, C₁-C₆-Alkyl, C₂-C₁₀-Alkenyl, C₂-C₁₀-Alkinyl, C₁-C₆-Halogenalkyl, C₂-C₁₀-Halogenalkenyl, C₁-C₆-Alkoxy, C₂-C₁₀-Alkenyloxy, C₂-C₁₀-Alkinyloxy, C₁-C₆-Halogenalkoxy, C₃-C₆-Cycloalkyl, C₃-C₆-Cycloalkenyl, C₃-C₆-Cycloalkoxy, C₁-C₈-Alkoxycarbonyl, C₂-C₁₀-Alkenyloxycarbonyl, C₂-C₁₀-Alkinyloxycarbonyl, Aminocarbonyl, C₁-C₈-Alkylaminocarbonyl, Di-(C₁-C₈-)alkylaminocarbonyl, C₁-C₈-Alkoximinoalkyl, C₂-C₁₀-Alkenyloximinoalkyl, C₂-C₁₀-Alkinyloximinoalkyl, C₁-C₈-Alkylcarbonyl, C₂-C₁₀-Alkenylcarbonyl, C₂-C₁₀-Alkinylcarbonyl, C₃-C₆-Cycloalkylcarbonyl, oder ein fünf- bis zehngliedriger gesättigter, partiell ungesättigter oder aromatischer Heterocyclus, enthaltend ein bis vier Heteroatome aus der Gruppe O, N oder S;
- X: SOₘ-R^{x}, NR^{x}R^{y} oder NR^{x-}(C=O) -R^{y};
R^{x}, R^{y}: Wasserstoff, C₁-C₆-Alkyl, C₂-C₁₀-Alkenyl, C₂-C₁₀-Alkinyl, C₃-C₆-Cycloalkyl, C₃-C₆-Cycloalkenyl, wobei die vorstehenden Reste partiell oder vollständig halogeniert sein können oder durch Cyan, C₁-C₄-Alkoximino,C₂-C₄-Alkenyloximino, C₂-C₄-Alkinyloximino oder C₁-C₄-Alkoxy substituiert sein können;
- m: 0 oder eine ganze Zahl 1 bis 3.

Außerdem betrifft die Erfindung Verfahren zur Herstellung dieser Verbindungen, sie enthaltende Mittel sowie ihre Verwendung zur Bekämpfung von Schadpilzen.

Aus EP-A 71 792, EP-A 550 113, WO-A 94/20501, EP-A 834 513, WO-A 98/46608 und WO-A 99/41255 sind 5-Chlortriazolopyrimidine zur Bekämpfung von Schadpilzen bekannt.

Ihre Wirkung ist jedoch in vielen Fällen nicht zufriedenstellend. Daher lag als Aufgabe zugrunde, Verbindungen mit verbesserter Wirksamkeit zu finden.

Demgemäß wurden die eingangs definierten Verbindungen gefunden. Desweiteren wurden Verfahren zu ihrer Herstellung, sie enthaltende Mittel sowie Verfahren zur Bekämpfung von Schadpilzen unter Verwendung der Verbindungen I gefunden.

Die Verbindungen der Formel I unterscheiden sich von den aus den oben genannten Schriften in der Kombination des 5-Alkylrestes mit über Kohlenstoff gebundenen Gruppen in der 7-Position.

Die Verbindungen der Formel I weisen eine gegenüber den bekannten Verbindungen erhöhte Wirksamkeit gegen Schadpilze auf.

Die Verbindungen I können auf verschiedenen Wegen erhalten werden; vorteilhaft geht man von 5-Aminotriazol der Formel II aus, das mit Dicarbonylverbindungen der Formel III kondensiert wird.

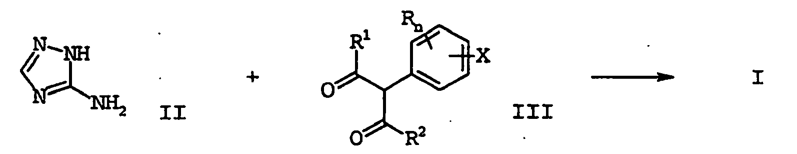

Diese Umsetzung erfolgt üblicherweise bei Temperaturen von 80°C bis 250°C, vorzugsweise 120°C bis 180°C, ohne Solvens oder in einem inerten organischen Lösungsmittel in Gegenwart einer Base [vgl. EP-A 770 615] oder in Gegenwart von Essigsäure unter den aus Adv. Het. Chem. Bd. 57, S. 81ff. (1993) bekannten Bedingungen.

Geeignete Lösungsmittel sind aliphatische Kohlenwasserstoffe, aromatische Kohlenwasserstoffe wie Toluol, o-, m- und p-Xylol, halogenierte Kohlenwasserstoffe, Ether, Nitrile, Ketone, Alkohole, sowie N-Methylpyrrolidon, Dimethylsulfoxid, Dimethylformamid und Dimethylacetamid. Besonders bevorzugt wird die Umsetzung ohne Lösungsmittel oder in Ethylenglykoldimethylether, Chlorbenzol, Xylol, Dimethylsulfoxid, N-Methylpyrrolidon durchgeführt. Es können auch Gemische der genannten Lösungsmittel verwendet werden.

Als Basen kommen allgemein anorganische Verbindungen wie Alkalimetall- und Erdalkalimetallhydroxide, Alkalimetall- und Erdalkalimetalloxide, Alkalimetall- und Erdalkalimetallhydride, Alkalimetallamide, Alkalimetall- und Erdalkalimetallcarbonate sowie Alkalimetallhydrogencarbonate, metallorganische Verbindungen, insbesondere Alkalimetallalkyle, Alkylmagnesiumhalogenide sowie Alkalimetall- und Erdalkalimetallalkoholate und Dimethoxymagnesium, außerdem organische Basen, z.B. tertiäre Amine wie Trimethylamin, Triethylamin, Di-isopropylethylamin, Tributylamin und N-Methylpiperidin, N-Methylmorpholin, Pyridin, substituierte Pyridine wie Collidin, Lutidin und 4-Dimethylaminopyridin sowie bicyclische Amine in Betracht. Besonders bevorzugt werden tertiäre Amine wie Di-isopropylethylamin, Tributylamin, N-Methylmorpholin oder N-Methylpiperidin.

Die Basen werden im allgemeinen in katalytischen Mengen eingesetzt, sie können aber auch äquimolar, im Überschuß oder gegebenenfalls als Lösungsmittel verwendet werden.

Die Edukte werden im allgemeinen in äquimolaren Mengen miteinander umgesetzt. Es kann für die Ausbeute vorteilhaft sein, die Base und das Diketon III in einem Überschuß bezogen auf II einzusetzen.

Die Diketone III lassen sich analog literaturbekannter Verfahren beispielsweise - wie in den zuvor genannten Schriften aufgeführt - herstellen. Die Diketone mit einem Acylaminosubstituenten lassen sich beispielsweise aus der entsprechenden Aminoverbindung durch Acylierung gewinnen. Die Aminogruppierung kann im allgemeinen durch Reduktion eines geeigneten Nitro-Vorläufers in den Phenylring eingeführt werden. Die Sülfonsäure-Gruppierung läßt sich durch direkte Sulfonierung mit Schwefelsäure oder Oleum eines geeigneten Vorläufers in den Phenylring einbringen. Die Sulfonsäuregruppierung kann jedoch auch durch Sandmeyer Reaktion mit Schwefeltrioxid aus einem geeigneten Diazoniumsalz aufgebaut werden. Das Diazoniumsalz läßt sich aus der obengenannten Aminoverbindung gewinnen. Die Sulfoxide und Sulfone können durch Oxidation der entsprechenden Alkylarylsulfide nach literaturbekannten Verfahren, beispielsweise mit Wasserstoffperoxid, Persäuren oder Selendioxid hergestellt werden.

Verbindungen der Formel I können auch durch Kupplung von 5-Halogentriazolopyrimidinen der Formel IV (Y steht für Halogen, insbesondere für Chlor oder Brom) mit metallorganischen Reagenzien der Formel VII erhalten werden.

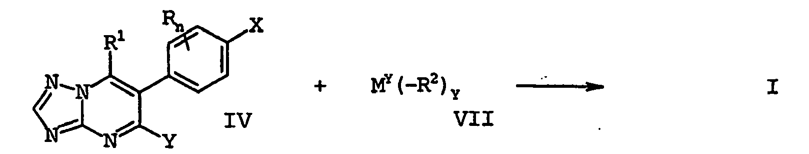

In Formel VII steht M für ein Metallion der Wertigkeit Y, wie beispielsweise B, Zn, Mg oder Sn. In einer Ausführungsform dieses Verfahrens erfolgt die Umsetzung unter Übergangsmetallkatalyse, wie Ni- oder Pd-Katalyse. Diese Reaktion kann beispielsweise analog folgender Methoden durchgeführt werden: J. Chem. Soc. Perkin Trans. 1, 1187 (1994), ebenda, 2345 (1996); WO-A 99/41255; Aust. J. Chem., Bd. 43, 733 (1990); J. Org. Chem., Bd. 43, 358 (1978); J. Chem. Soc. Chem. Commun. 866 (1979); Tetrahedron Lett., Bd. 34, 8267 (1993); ebenda, Bd. 33, 413 (1992). Insbesondere wenn M für Zn oder Mg steht kann die Reaktion auch ohne Katalysator durchgeführt werden. Die Verbindungen IV sind aus den eingangs zitierten Schriften bekannt. Insbesondere werden sie aus 5,7-Dichlortriazolopyrimidinen gewonnen, indem der Rest R¹ mittels metallorganischer Verfahren ähnlich wie zuvor beschrieben eingeführt wird.

Die erfindungsgemäßen Verbindungen der Formel I' sind auch zugänglich durch Umsetzung von 5-Halogentriazolopyrimidinen der Formel IV mit substituierten Malonsäureestern der Formel V, in der R^{x} für C₁-C₄-Alkyl, Allyl, Phenyl oder Benzyl steht, anschließender Verseifung des entstandenen Esters VI und Decarboxylierung der Carbonsäure VIa.

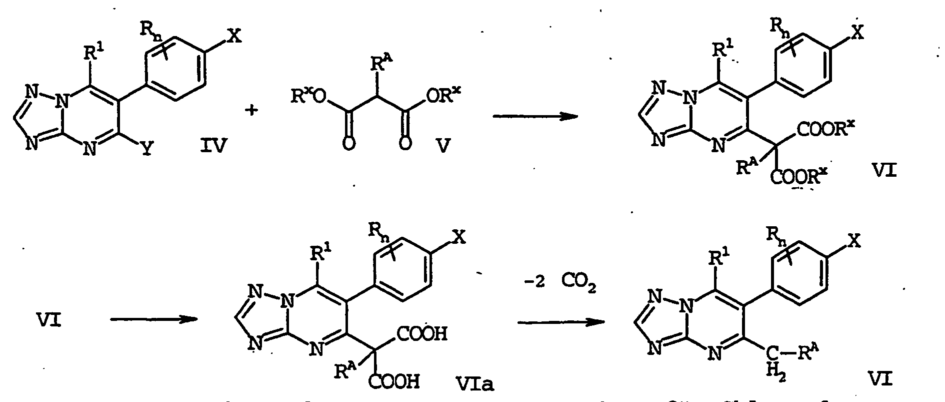

In Formel IV steht Y für Halogen, insbesondere für Chlor oder Brom. Die Verbindungen IV sind aus den eingangs zitierten Schriften bekannt. In Formel I' haben n, R und R¹ die für Formel I definierte Bedeutung und R^{A} steht für Wasserstoff oder C₁-C₃-Alkyl, das durch Halogen, Cyano, Nitro oder C₁-C₂-Alkoxy substituiert sein kann.

In einer bevorzugten Ausführungsform des erfindungsgemäßen Verfahrens bedeutet R^{A} Wasserstoff oder Methyl, insbesondere Wasserstoff.

Die Ausgangsstoffe V sind in der Literatur bekannt [J. Am. Chem. Soc., Bd. 64, 2714 (1942); J. Org. Chem., Bd. 39, 2172 (1974); Helv. Chim. Acta, Bd. 61, 1565 (1978)] oder können gemäß der zitierten Literatur hergestellt werden.

Die anschließende Spaltung des Esters erfolgt unter den allgemein üblichen Bedingungen [vgl.: Greene & Wuts, Protective Groups in Organic Synthesis, Wiley (1991), S. 224 ff: Spaltung von Alkylestern unter Pd-Katalyse (S. 248); hydrierende Spaltung von Benzylestern (S. 251); Spaltung von Methyl- bzw. Ethylestern in Gegenwart von Lithiumsalzen, wie LiI (S.232), LiBr oder LiCl; oder unter sauren oder alkalischen Bedingungen]. In Abhängigkeit der Strukturelemente R^{A}, Rₙ und R¹ kann die alkalische oder die saure Verseifung der Verbindungen VI vorteilhaft sein. Unter den Bedingungen der Esterverseifung kann die Decarboxylierung zu I' bereits ganz oder teilweise erfolgen.

Die Decarboxylierung erfolgt üblicherweise bei Temperaturen von 20°C bis 180°C, vorzugsweise 50°C bis 120°C, in einem inerten Lösungsmittel, gegebenenfalls in Gegenwart einer Säure.

Geeignete Säuren sind Salzsäure, Schwefelsäure, Phosphorsäure, Ameisensäure, Essigsäure, p-Toluolsulfonsäure. Geeignete Lösungsmittel sind Wasser, aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol, sowie Dimethylsulfoxid, Dimethylformamid und Dimethylacetamid, besonders bevorzugt wird die Reaktion in Salzsäure oder Essigsäure durchgeführt. Es können auch Gemische der genannten Lösungsmittel verwendet werden.

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischen- und Endprodukte fallen z.T. in Form farbloser oder schwach bräunlicher, zäher Öle an, die unter vermindertem Druck und bei mäßig erhöhter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.

Sofern einzelne Verbindungen I nicht auf den voranstehend beschriebenen Wegen zugänglich sind, können sie durch Derivatisierung anderer Verbindungen I hergestellt werden.

Sofern bei der Synthese Isomerengemische anfallen, ist im allgemeinen jedoch eine Trennung nicht unbedingt erforderlich, da sich die einzelnen Isomere teilweise während der Aufbereitung für die Anwendung oder bei der Anwendung (z.B. unter Licht-, Säure- oder Baseneinwirkung) ineinander umwandeln können. Entsprechende Umwandlungen können auch nach der Anwendung, beispielsweise bei der Behandlung von Pflanzen in der behandelten Pflanze oder im zu bekämpfenden Schadpilz oder tierischen Schädling erfolgen.

Bei den in den vorstehenden Formeln angegebenen Definitionen der Symbole wurden Sammelbegriffe verwendet, die allgemein repräsentativ für die folgenden Substituenten stehen:
**Halogen:** Fluor, Chlor, Brom und Jod;
**Alkyl:** gesättigte, geradkettige oder verzweigte Kohlenwasserstoffreste mit 1 bis 4, 6, 8 oder 10 Kohlenstoffatomen, z.B. C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;
**Halogenalkyl:** geradkettige oder verzweigte Alkylgruppen mit 1 bis 10 Kohlenstoffatomen (wie vorstehend genannt), wobei in diesen Gruppen teilweise oder vollständig die Wasserstoffatome durch Halogenatome wie vorstehend genannt ersetzt sein können, z.B. C₁-C₂-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
**Alkenyl:** ungesättigte, geradkettige oder verzweigte Kohlenwasserstoffreste mit 2 bis 4, 6, 8 oder 10 Kohlenstoffatomen und einer Doppelbindung in einer beliebigen Position, z.B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-lpropenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-lpropenyl und 1-Ethyl-2-methyl-2-propenyl;
**Halogenalkenyl:** ungesättigte, geradkettige oder verzweigte Kohlenwasserstoffreste mit 2 bis 10 Kohlenstoffatomen und einer Doppelbindung in einer beliebigen Position (wie vorstehend genannt), wobei in diesen Gruppen die Wasserstoffatome teilweise oder vollständig gegen Halogenatome wie vorstehend genannt, insbesondere Fluor, Chlor und Brom, ersetzt sein können;
**Alkinyl:** geradkettige oder verzweigte Kohlenwasserstoffgruppen mit 2 bis 4, 6, 8 oder 10 Kohlenstoffatomen und einer Dreifachbindung in einer beliebigen Position, z.B. C₂-C₆-Alkinyl wie Ethinyl, 1-Propinyl, 2-Propinyl, 1-Butinyl, 2-Butinyl, 3-Butinyl-, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl;
**Cycloalkyl:** mono- oder bicyclische, gesättigte Kohlenwasserstoffgruppen mit 3 bis 6, 8, 10 oder 12 Kohlenstoffringgliedern, z.B. C₃-C₈-Cycloalkyl wie Cyclopropyl, Cyclobutyl, Cyclopentyl, Cyclohexyl, Cycloheptyl und Cyclooctyl, oder C₇-C₁₂-Bicycloalkyl;
**Aryl:** ein ein- bis dreikerniges aromatisches Ringsystem enthaltend 6 bis 14 Kohlenstoffringglieder, z.B. Phenyl, Naphthyl und Anthracenyl;
**fünf- bis zehngliedriger gesättigter, partiell ungesättigter oder aromatischer Heterocyclus, der über Kohlenstoff mit dem Triazolopyrimidin verbunden ist und ein bis vier Heteroatome aus der Gruppe O, N oder S enthält, :**
   - **5- oder 6-gliedriges Heterocyclyl,** enthaltend ein bis drei Stickstoffatome und/oder ein Sauerstoff- oder Schwefelatom oder ein oder zwei Sauerstoff- und/oder Schwefelatome, z.B. 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,4-Dihydrofur-2-yl, 2,4-Dihydrofur-3-yl, 2,3-Dihydrothien-2-yl, 2,3-Dihydrothien-3-yl, 2,4-Dihydrothien-2-yl, 2,4-Dihydrothien-3-yl, 2-Pyrrolin-2-yl, 2-Pyrrolin-3-yl, 3-Pyrrolin-2-yl, 3-Pyrrolin-3-yl, 2-Isoxazolin-3-yl, 3-Isoxazolin-3-yl, 4-Isoxazolin-3-yl, 2-Isoxazolin-4-yl, 3-Isoxazolin-4-yl, 4-Isoxazolin-4-yl, 2-Isoxazolin-5-yl, 3-Isoxazolin-5-yl, 4-Isoxazolin-5-yl, 2-Isothiazolin-3-yl, 3-Isothiazolin-3-yl, 4-Isothiazolin-3-yl, 2-Isothiazolin-4-yl, 3-Isothiazolin-4-yl, 4-Isothiazolin-4-yl, 2-Isothiazolin-5-yl, 3-Isothiazolin-5-yl, 4-Isothiazolin-5-yl, 2,3-Dihydropyrazol-1-yl, 2,3-Dihydropyrazol-2-yl, 2,3-Dihydropyrazol-3-yl, 2,3-Dihydropyrazol-4-yl, 2,3-Dihydropyrazol-5-yl, 3,4-Dihydropyrazol-1-yl, 3,4-Dihydropyrazol-3-yl, 3,4-Dihydropyrazol-4-yl, 3,4-Dihydropyrazol-5-yl, 4,5-Dihydropyrazol-1-yl, 4,5-Dihydropyrazol-3-yl, 4,5-Dihydropyrazol-4-yl, 4,5-Dihydropyrazol-5-yl, 2,3-Dihydrooxazol-2-yl, 2,3-Dihydrooxazol-3-yl, 2,3-Dihydrooxazol-4-yl, 2,3-Dihydrooxazol-5-yl, 3,4-Dihydrooxazol-2-yl, 3,4-Dihydrooxazol-3-yl, 3,4-Dihydrooxazol-4-yl, 3,4-Dihydrooxazol-5-yl, 3,4-Dihydrooxazol-2-yl, 3,4-Dihydrooxazol-3-yl, 3,4-Dihydrooxazol-4-yl, 2-Piperidinyl, 3-Piperidinyl, 4-Piperidinyl, 1,3-Dioxan-5-yl, 2-Tetrahydropyranyl, 4-Tetrahydropyranyl, 2-Tetrahydrothienyl, 3-Hexahydropyridazinyl, 4-Hexahydropyridazinyl, 2-Hexahydropyrimidinyl, 4-Hexahydropyrimidinyl, 5-Hexahydropyrimidinyl, 2-Piperazinyl, 1,3,5-Hexahydro-triazin-2-yl und 1,2,4-Hexahydrotriazin-3-yl;
   - **5-gliedriges Heteroaryl,** enthaltend ein bis vier Stickstoffatome oder ein bis drei Stickstoffatome und ein Schwefel- oder Sauerstoffatom: 5-Ring Heteroarylgruppen, welche neben Kohlenstoffatomen ein bis vier Stickstoffatome oder ein bis drei Stickstoffatome und ein Schwefel- oder Sauerstoffatom als Ringglieder enthalten können, z.B. 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl und 1,3,4-Triazol-2-yl;
   - **benzokondensiertes 5-gliedriges Heteroaryl,** enthaltend ein bis drei Stickstoffatome oder ein Stickstoffatom und ein Sauerstoff- oder Schwefelatom: 5-Ring Heteroarylgruppen, welche neben Kohlenstoffatomen ein bis vier Stickstoffatome oder ein bis drei.Stickstoffatome und ein Schwefel- oder Sauerstoffatom als Ringglieder enthalten können, und in welchen zwei benachbarte Kohlenstoffringglieder oder ein Stickstoff- und ein benachbartes Kohlenstoffringglied durch eine Buta-1,3-dien-1,4-diylgruppe verbrückt sein können;
   - **6-gliedriges Heteroaryl,** enthaltend ein bis drei bzw. ein bis vier Stickstoffatome: 6-Ring Heteroarylgruppen, welche neben Kohlenstoffatomen ein bis drei bzw. ein bis vier Stickstoffatome als Ringglieder enthalten können, z.B. 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl und 1,2,4-Triazin-3-yl.

In dem Umfang der vorliegenden Erfindung sind die (R)- und (S)-Isomere und die Razemate von Verbindungen der Formel I eingeschlossen, die chirale Zentren aufweisen.

Im Hinblick auf ihre bestimmungsgemäße Verwendung der Triazolopyrimidine der Formel I sind die folgenden Bedeutungen der Substituenten, und zwar jeweils für sich allein oder in Kombination, besonders bevorzugt:

Verbindungen I werden bevorzugt, in denen R¹ für C₃-C₈-Alkyl, C₃-C₈-Alkenyl, C₃-C₈-Alkinyl, C₃-C₆-Cycloalkyl oder C₅-C₆-Cycloalkenyl steht.

Insbesondere werden Verbindungen I bevorzugt, in denen R¹ für C₁-C₈-Alkyl oder C₁-C₆-Halogenalkyl steht.

Daneben werden Verbindungen I bevorzugt, in denen R¹ für C₂-C₁₀-Alkinyl und insbesondere für C₂-C₁₀-Alkenyl steht. Besonders bevorzugt sind verzweigtes C₂-C₁₀-Alkenyl.

Gleichermaßen bevorzugt sind Verbindungen I, in denen R¹ für einen 5- oder 6-gliedrigen gesättigten oder aromatischen Heterocyclus steht, der über Kohlenstoff gebunden ist.

Außerdem werden Verbindungen I besonders bevorzugt, in denen R¹ für C₃-C₆-Cycloalkyl oder für C₅-C₆-Cycloalkyl steht, welche durch C₁-C₄-Alkyl substituiert sein können.

Verbindungen I werden besonders bevorzugt, in denen R^{a} für Halogen, Cyano, C₁-C₆-Alkyl, C₂-C₆-Alkenyl, C₂-C₆-Alkinyl, C₁-C₆-Alkoxy, C₁-C₆-Alkoxycarbonyl, C₁-C₆-Alkoximino, C₂-C₆-Alkenyloximino, C₂-C₆-Alkinyloximino, C₃-C₆-Cycloalkyl oder C₅-C₆-Cycloalkenyl steht, wobei die aliphatischen oder alicyclischen Gruppen ihrerseits partiell oder vollständig halogeniert sein oder eine bis drei Gruppen R^{b} tragen können.

Insbesondere werden Verbindungen I bevorzugt, in denen R^{b} für Halogen, Cyano, C₁-C₆-Alkyl, C₂-C₆-Alkenyl, C₂-C₆-Alkinyl, C₁-C₆-Alkylcarbonyl, C₁-C₆-Haloalkylcarbonyl oder C₁-C₆-Alkoxy steht.

Besonders bevorzugt werden auch Verbindungen I, in denen R² C₁-C₄-Alkyl bedeutet, das durch Halogen substituiert sein kann.

Gleichermaßen besonders bevorzugt sind Verbindungen I, in denen R² für Methyl steht.

Daneben werden Verbindungen I besonders bevorzugt, in denen R² für Halogenmethyl steht.

Insbesondere werden auch Verbindungen I bevorzugt, in denen ein Substituent R in 2-Position steht und n eine ganze Zahl von 1 bis 3, insbesondere 1 oder 2, bedeutet.

Außerdem werden Verbindungen I besonders bevorzugt, in denen n 2 oder 3 bedeutet und ein Substituent R in 2-Position steht.

Weiterhin werden Verbindungen I bevorzugt, in denen R für Fluor, Chlor, Brom, Cyano, C₁-C₆-Alkyl oder C₁-C₆-Alkoxy steht.

Gleichermaßen besonders bevorzugt sind Verbindungen I, in denen R für Fluor, Chlor, Methyl, Trifluormethyl oder Methoxy steht.

Daneben werden Verbindungen I besonders bevorzugt, in denen Rₙ für 2-Chlor, 2-Fluor, 2-Methyl, 2-Methoxy, 2-Trifluormethyl; 2-Trifluormethyl, 6-chlor, 2-Chlor, 6-fluor, 2,6-Difluor, 2-Fluor, 6-methyl, 2,4-Difluor, 2-Fluor, 4-chlor, 2-Fluor, 3-methyl, 2-Fluor, 4-methyl, 2-Chlor, 4-fluor, 2,4-Dichlor, 2-Chlor-4-methyl, 2-Chlor-3-methyl, 2,6-Dichlor, 2-Chlor-6-methyl, 2-Methyl, 4-fluor, 2-Methyl, 4-chlor, 2,4-Dimethyl, 2,3-Dimethyl, 2-Methyl, 6-fluor, 2-Methyl,6-Chlor oder 2,6-Dimethyl steht.

Außerdem werden Verbindungen I besonders bevorzugt, in denen X C₁-C₆-Alkyl-sulfonyl, C₁-C₆-Alkyl-sulfenyl, C₁-C₆-Alkyl-sulfoxyl, C₁-C₆-Alkyl-mercapto, Amino, C₁-C₆-Alkylamino, Di-(C₁-C₆-Alkyl)amino, C₁-C₆-Alkylcarbonylamino, C₁-C₆-Alkylcarbonyl(C₁-C₆-Alkyl)amino.

Es werden Verbindungen I bevorzugt, in denen der Substituent X in 3- oder 5-Stellung am Phenylring sitzt.

Insbesondere werden Verbindungen I besonders bevorzugt, in denen der Substituent X in 4-Stellung am Phenylring sitzt.

Gleichermaßen besonders bevorzugt sind Verbindungen I, in denen m 1 oder 2 bedeutet. Das Schwefelatom ist vorzugsweise direkt an den Phenylring gebunden. Wenn m 2 oder 3 bedeutet, kann der Schwefel auch über Sauerstoff an den Phenylring gebunden sein.

### Triazolopyrimidine der Formel I'

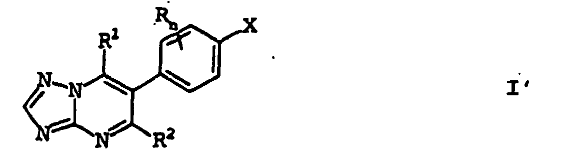

in der der Index und die Substituenten folgende Bedeutung haben:
- R¹: C₃-C₈-Alkyl, C₃-C₉-Alkenyl, C₃-C₈-Alkinyl, C₃-C₆-Cycloalkyl, C₅-C₆-Cycloalkenyl; wobei R¹ partiell oder vollständig halogeniert oder durch eine bis vier gleiche oder verschiedene Gruppen R^{a} substituiert sein kann:
R^{a} Halogen, Cyano, C₁-C₆-Alkyl, C₂-C₆-Alkenyl, C₂-C₆-Alkinyl, C₁-C₆-Alkoxy, C₁-C₆-Alkoxycarbonyl, C₁-C₆-Alkoximino, C₂-C₆-Alkenyloximino, C₂-C₆-Alkinyloximino;
- R²: C₁-C₄-Alkyl, das durch Halogen substituiert sein kann.
- n: eine ganze Zahl von 0 bis 2;
- R: Fluor, Chlor, Brom, Cyano, C₁-C₆-Alkyl, C₁-C₆-Alkoxy;
- X: SO-R^{x}, SO₂-R^{x} oder NR^{x}-(C=O)-R^{y};
R^{x}, R^{y}: Wasserstoff, C₁-C₆-Alkyl, C₂-C₆-Alkenyl oder C₃-C₆-Cycloalkyl, wobei die vorstehenden Reste partiell oder vollständig halogeniert sein können sind besonders bevorzugt;

Die Verbindungen I eignen sich als Fungizide. Sie zeichnen sich aus durch eine hervorragende Wirksamkeit gegen ein breites Spektrum von pflanzenpathogenen Pilzen, insbesondere aus der Klasse der *Ascomyceten, Deuteromyceten, Oomyceten* und *Basidiomyceten.* Sie sind zum Teil systemisch wirksam und können im Pflanzenschutz als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Bananen, Baumwolle, Soja, Kaffee, Zuckerrohr, Wein, Obst- und Zierpflanzen und Gemüsepflanzen wie Gurken, Bohnen, Tomaten, Kartoffeln und Kürbisgewächsen, sowie an den Samen dieser Pflanzen.

Speziell eignen sie sich zur Bekämpfung folgender Pflanzenkrankheiten:
- *Alternaria-*Arten an Gemüse und Obst,
- *Bipolaris-* und *Drechslera-*Arten an Getreide, Reis und Rasen,
- *Blumeria graminis* (echter Mehltau) an Getreide,
- *Botrytis cinerea* (Grauschimmel) an Erdbeeren, Gemüse, Zierpflanzen und Reben,
- *Erysiphe cichoracearum* und *Sphaerotheca fuliginea* an Kürbisgewächsen,
- Fusarium- und *Verticillium*-Arten an verschiedenen Pflanzen,
- *Mycosphaerella-*Arten an Getreide, Bananen und Erdnüssen,
- *Phytophthora infestans* an Kartoffeln und Tomaten,
- *Plasmopara viticola* an Reben,
- *Podosphaera leucotricha* an Äpfeln,
- *Pseudocercosporella herpotrichoides* an Weizen und Gerste,
- *Pseudoperonospora*-Arten an Hopfen und Gurken,
- *Puccinia*-Arten an Getreide,
- *Pyricularia oryzae* an Reis,
- *Rhizoctonia*-Arten an Baumwolle, Reis und Rasen,
- *Septoria tritici* und *Stagonospora nodorum* an Weizen,
- *Uncinula* necator an Reben,
- *Ustilago*-Arten an Getreide und Zuckerrohr, sowie
- *Venturia*-Arten (Schorf) an Äpfeln und Birnen.

Die Verbindungen I eignen sich außerdem zur Bekämpfung von Schadpilzen wie *Paecilomyces variotii* im Materialschutz (z.B. Holz, Papier, Dispersionen für den Anstrich, Fasern bzw. Gewebe) und im Vorratsschutz.

Die Verbindungen I werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung kann sowohl vor als auch nach der Infektion der Materialien, Pflanzen oder Samen durch die Pilze erfolgen.

Die fungiziden Mittel enthalten im allgemeinen zwischen 0,1 und 95, vorzugsweise zwischen 0,5 und 90 Gew.-% Wirkstoff.

Die Aufwandmengen liegen bei der Anwendung im Pflanzenschutz je nach Art des gewünschten Effektes zwischen 0,01 und 2,0 kg Wirkstoff pro ha.

Bei der Saatgutbehandlung werden im allgemeinen Wirkstoffmengen von 0,001 bis 0,1 g, vorzugsweise 0,01 bis 0,05 g je Kilogramm Saatgut benötigt.

Bei der Anwendung im Material- bzw. Vorratsschutz richtet sich die Aufwandmenge an Wirkstoff nach der Art des Einsatzgebietes und des gewünschten Effekts. Übliche Aufwandmengen sind im Materialschutz beispielsweise 0,001 g bis 2 kg, vorzugsweise 0,005 g bis 1 kg Wirkstoff pro Qubikmeter behandelten Materials.

Die Verbindungen I können in die üblichen Formulierungen überführt werden, z.B. Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung der erfindungsgemäßen Verbindung gewährleisten.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln. Als Lösungsmittel / Hilfsstoffe kommen dafür im wesentlichen in Betracht:
- Wasser, aromatische Lösungsmittel (z.B. Solvesso Produkte, Xylol), Paraffine (z.B. Erdölfraktionen), Alkohole (z.B. Methanol, Butanol, Pentanol, Benzylalkohol), Ketone (z.B. Cyclohexanon, gamma-Butryolacton), Pyrrolidone (NMP, NOP), Acetate (Glykoldiacetat), Glykole, Dimethylfettsäureamide, Fettsäuren und Fettsäureester. Grundsätzlich können auch Lösungsmittelgemische verwendet werden,
- Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin-Sulfitablaugen und Methylcellulose.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate, Fettsäuren und sulfatierte Fettalkoholglykolether zum Einsatz, ferner Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Tristerylphenylpolyglykolether, Alkyl-arylpolyetheralkohole, Alkohol- und Fettalkoholethylenoxid-Kondensate, ethoxyliertes Rizinusöl, Polyoxyethylenalkylether, ethoxyliertes Polyoxypropylen, Laurylalkoholpoly-glykoletheracetal, Sorbitester, Ligninsulfitablaugen und Methylcellulose in Betracht.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B.

Toluol, Xylol, Paraffin, Tetrahydronaphthalin, alkylierte Naphthaline oder deren Derivate, Methanol, Ethanol, Propanol, Butanol, Cyclohexanol, Cyclohexanon, Isophoron, stark polare Lösungsmittel, z.B. Dimethylsulfoxid, N-Methylpyrrolidon oder Wasser in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und-Homogengranulate, können durch Bindung der Wirkstoffe an feste Trägerstoffe hergestellt werden. Feste Trägerstoffe sind z.B. Mineralerden, wie Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,01 und 95 Gew.-%, vorzugsweise zwischen 0,1 und 90 Gew.-% des Wirkstoffs. Die Wirkstoffe werden dabei in einer Reinheit von 90% bis 100%, vorzugsweise 95% bis 100% (nach NMR-Spektrum) eingesetzt.

### Beispiele für Formulierungen sind: 1. Produkte zur Verdünnung in Wasser

A) Wasserlösliche Konzentrate (SL)
   10 Gew.-Teile einer erfindungsgemäßen Verbindung werden in Wasser oder einem wasserlöslichen Lösungsmittel gelöst. Alternativ werden Netzmittel oder andere Hilfsmittel zugefügt. Bei der Verdünnung in Wasser löst sich der Wirkstoff.
B) Dispergierbare Konzentrate (DC)
   20 Gew.-Teile einer erfindungsgemäßen Verbindung werden in Cyclohexanon unter Zusatz eines Dispergiermittels z.B. Polyvinylpyrrolidon gelöst. Bei Verdünnung in Wasser ergibt sich eine Dispersion.
C) Emulgierbare Konzentrate (EC)
   15 Gew.-Teile einer erfindungsgemäßen Verbindung werden in Xylol unter Zusatz von Ca-Dodecylbenzolsulfonat und Ricinusölethoxylat (jeweils 5 %) gelöst. Bei der Verdünnung in Wasser ergibt sich eine Emulsion.
D) Emulsionen (EW, EO)
   40 Gew.-Teile einer erfindungsgemäßen Verbindung werden in Xylol unter Zusatz von Ca-Dodecylbenzolsulfonat und Ricinusölethoxylat (jeweils 5 %) gelöst. Diese Mischung wird mittels einer Emulgiermaschine (Ultraturax) in Wasser eingebracht und zu einer homogenen Emulsion gebracht. Bei der Verdünnung in Wasser ergibt sich eine Emulsion.
E) Suspensionen (SC, OD)
   20 Gew.-Teile einer erfindungsgemäßen Verbindung werden unter Zusatz von Dispergier- und Netzmitteln und Wasser oder einem organischen Lösungsmittel in einer Rührwerkskugelmühle zu einer feinen Wirkstoffsuspension zerkleinert. Bei der Verdünnung in Wasser ergibt sich eine stabile Suspension des Wirkstoffs.
F) Wasserdispergierbare und wasserlösliche Granulate (WG, SG)
   - 50 Gew.-Teile einer erfindungsgemäßen Verbindung werden unter Zusatz von Dispergier- und Netzmitteln fein gemahlen und mittels technischer Geräte (z.B. Extrusion, Sprühturm, Wirbelschicht) als wasserdispergierbare oder wasserlösliche Granulate hergestellt. Bei der Verdünnung in Wasser ergibt sich eine stabile Dispersion oder Lösung des Wirkstoffs.
G) Wasserdispergierbare und wasserlösliche Pulver (WP, SP)
   75 Gew.-Teile einer erfindungsgemäßen Verbindung werden unter Zusatz von Dispergier- und Netzmitteln sowie Kieselsäuregel in einer Rotor-Strator Mühle vermahlen. Bei der Verdünnung in Wasser ergibt sich eine stabile Dispersion oder Lösung des Wirkstoffs.

### 2. Produkte für die Direktapplikation

H) Stäube (DP)
   5 Gew.Teile einer erfindungsgemäßen Verbindung werden fein gemahlen und mit 95 % feinteiligem Kaolin innig vermischt. Man erhält dadurch ein Stäubemittel.
I) Granulate (GR, FG, GG, MG)
   0.5 Gew-Teile einer erfindungsgemäßen Verbindung werden fein gemahlen und mit 95.5 % Trägerstoffe verbunden. Gängige Verfahren sind dabei die Extrusion, die Sprühtrocknung oder die Wirbelschicht. Man erhält dadurch ein Granulat für die Direktapplikation.
J) ULV- Lösungen (UL)
   10 Gew.-Teile einer erfindungsgemäßen Verbindung werden in einem organischen Lösungsmittel z.B. Xylol gelöst. Dadurch erhält man ein Produkt für die Direktapplikation.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Wässrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Die Wirkstoffkonzentrationen in den anwendungsfertigen Zubereitungen können in größeren Bereichen variiert werden. Im allgemeinen liegen sie zwischen 0,0001 und 10%, vorzugsweise zwischen 0,01 und 1%.

Die Wirkstoffe können auch mit gutem Erfolg im Ultra-Low-Volume-Verfahren (ULV) verwendet werden, wobei es möglich ist, Formulierungen mit mehr. als 95 Gew.-% Wirkstoff oder sogar den Wirkstoff ohne Zusätze auszubringen.

Zu den Wirkstoffen können Öle verschiedenen Typs, Netzmittel, Adjuvants, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln. Beim Vermischen der Verbindungen I bzw. der sie enthaltenden Mittel in der Anwendungsform als Fungizide mit anderen Fungiziden erhält man in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
- Acylalanine wie Benalaxyl, Metalaxyl, Ofurace, Oxadixyl,
- Aminderivate wie Aldimorph, Dodine, Dodemorph, Fenpropimorph, Fenpropidin, Guazatine, Iminoctadine, Spiroxamin, Tridemorph
- Anilinopyrimidine wie Pyrimethanil, Mepanipyrim oder Cyrodinyl,
- Antibiotika wie Cycloheximid, Griseofulvin, Kasugamycin, Natamycin, Polyoxin oder Streptomycin,
- Azole wie Bitertanol, Bromoconazol, Cyproconazol, Difenoconazole, Dinitroconazol, Epoxiconazol, Fenbuconazol, Fluquiconazol, Flusilazol., Hexaconazol, Imazalil, Metconazol, Myclobutanil, Penconazol, Propiconazol, Prochloraz, Prothioconazol, Tebuconazol, Triadimefon, Triadimenol, Triflumizol, Triticonazol,
- Dicarboximide wie Iprodion, Myclozolin, Procymidon, Vinclozolin,
- Dithiocarbamate wie Ferbam, Nabam, Maneb, Mancozeb, Metam, Metiram, Propineb, Polycarbamat, Thiram, Ziram, Zineb,
- Heterocylische Verbindungen wie Anilazin, Benomyl, Boscalid, Carbendazim, Carboxin, Oxycarboxin, Cyazofamid, Dazomet, Dithianon, Famoxadon, Fenamidon, Fenarimol, Fuberidazol, Flutolanil, Furametpyr, Isoprothiolan, Mepronil, Nuarimol, Probenazol, Proquinazid, Pyrifenox, Pyroquilon, Quinoxyfen, Silthiofam, Thiabendazol, Thifluzamid, Thiophanat-methyl, Tiadinil, Tricyclazol, Triforine,
- Kupferfungizide wie Bordeaux Brühe, Kupferacetat, Kupferoxychlorid, basisches Kupfersulfat,
- Nitrophenylderivate, wie Binapacryl, Dinocap, Dinobuton, Nitrophthal-isopropyl
- Phenylpyrrole wie Fenpiclonil oder Fludioxonil,
- Schwefel
- Sonstige Fungizide wie Acibenzolar-S-methyl, Benthiavalicarb, Carpropamid, Chlorothalonil, Cyflufenamid, Cymoxanil, Dazomet, Diclomezin, Diclocymet, Diethofencarb, Edifenphos, Ethaboxam, Fenhexamid, Fentin-Acetat, Fenoxanil, Ferimzone, Fluazinam, Fosetyl, Fosetyl-Aluminium, Iprovalicarb, Hexachlorbenzol, Metrafenon, Pencycuron, Propamocarb, Phthalid, Toloclofos-methyl, Quintozene, Zoxamid
- Strobilurine wie Azoxystrobin, Dimoxystrobin, Fluoxastrobin, Kresoxim-methyl, Metominostrobin, Orysastrobin, Picoxystrobin, Pyraclostrobin oder Trifloxystrobin,
- Sulfensäurederivate wie Captafol, Captan, Dichlofluanid, Folpet, Tolylfluanid
- Zimtsäureamide und Analoge wie Dimethomorph, Flumetover oder Flumorph.

### Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in den anschließenden Tabellen mit physikalischen Angaben aufgeführt.

### Beispiel 1: Herstellung von 5-Methyl-6-(2-chlor-4-acetylaminophenyl)-7-(2-methylbutyl)-1,2,4-triazolo[1,5a]pyrimidin (I-16)

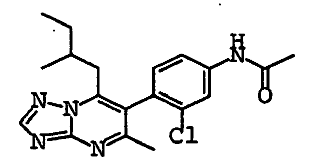

### 1.1. 5-Methyl-6-(2-chlor-4-amino-phenyl)-7-(2-methylbutyl)-1,2,4-triazolo[1,5a]pyrimidin

Eine Mischung von 3 g (8,3 mmol) 5-Methyl-6-(2-chlor-4-nitro-phenyl)-7- (2-methylbutyl)-1,2,4-triazolo[1,5a]pyrimidin (Herstellung analog WO 03/004465), 100 ml Essigsäure, 1 ml konz. Schwefelsäure und 0,5 g 10 % iger Palladiumkohle wurde über Nacht unter einer Wasserstoffatmosphäre gerührt.

Anschließend saugte man die Reaktionsmischung über Kieselgur ab, verdünnte die Essigsäurephase mit Wasser und extrahierte die wässrige Phase dreimal mit Methylenchlorid. Die vereinigten organischen Phasen wurden mit NaHCO₃-Lsg. und Wasser neutral gewaschen und eingeengt. Der Rückstand wurde säulenchromatographisch mit Cyclohexan/Essigester-Gemischen gereinigt.

### Man erhielt 2,1 g (80 %) der Titelverbindung 1.1. als farblosen Festkörper.

¹H-NMR (CDCl₃, δ in ppm): 8,45 (s, 1H); 7,0 (d, breit, 1H); 6,9 (s, breit, 1H); 6,7 (d, breit, 1H); 3,6 - 4,1 (s, sehr breit, 2 H); 3,1 (dd, 0,5 H); 2,95 (dd, 0,5 H); 2,85 (dd, 0,5 H); 2,7 (dd, 0,5 H); 2,4 (s, 3H), 2,1 (m, 1H); 1,0 - 1,4 (m, 2H); 0,7 - 0,85 (m, 6H)

### 1.2. 5-Methyl-6-(2-chlor-4-acetylamino-phenyl)-7-(2-methylbutyl)-1,2,4-triazvlo[1,5a]pyrimidin

Eine Mischung von 0,5 g (1,5 nmol) 5-Methyl-6-(2-chlor-4-aminophenyl)- 7-(2-methylbutyl)-1,2,4-triazolo[1,5a]pyrimidin (Beispiel 1.1.) und 10 ml Methylenchlorid wurde mit 0,25 g (3 mmol) Pyridin und 0,2 g (2,5 mmol) Acetylchlorid versetzt und ca. 1 Stunde bei Raumtemperatur gerührt.

Anschließend wurde die Reaktionsmischung mit verdünnter Salzsäure und Wasser gewaschen und eingeengt. Als Rückstand erhielt man 0,5 g (88 %) der Titelverbindung 1.2.

¹H-NMR (CDCl₃, 8 in ppm): 8,45 (s, 1H); 8,15 (s, breit, 1H); 7,95 (s, breit, 1H); 7,65 (m, 1H); 7,2 (m, 1H); 3,1 (dd, 0,5 H); 2,95 (dd, 0,5 H); 2,85 (dd, 0,5 H); 2,65 (dd, 0,5 H); 2,4 (s, 3H); 2,3 (s, 3H); 2,1 (m, 1H); 1,0 - 1,35 (m, 1H); 0,7 - 0,85 (m, 6H)

### Beispiel 2: 5-Methyl-6-(2,6-difluor-4-benzylthio-phenyl)-7-(2-methylbutyl)-1,2,4-triazolo[1,5a]pyrimidin (I-3)

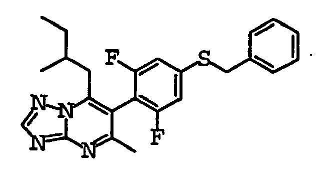

1,3 g (10 mmol) Benzylmercaptan in 50 ml N-Methylpyrrolidon wurden unter einer Stickstoffatmosphäre mit 0,3 g (12,5 mmol) Natriumhydrid versetzt und bis zur Beendigung der Wasserstoffentwicklung (ca. 15 min) bei Raumtemperatur gerührt.

Anschließend gab man 3,3 g (10 mmol) 5-Methyl-6-(2,4,6-trifluorphenyl)-7-(2-methylbutyl)-1,2,4-triazolo[1,5a]pyrimidin (Herstellung analog WO 03/004465) hinzu und rührte ca. 2 Stunden bei Raumtemperatur. Dann verdünnte man die Reaktionsmischung mit Wasser und extrahierte die wässrige Phase dreimal mit Methyl-t-butylether. Die vereinigten organischen Phasen wurden zweimal mit Wasser gewaschen und eingeengt. Der erhaltene Rückstand wurde mittels MPLC an Kieselgel RP-18 mit Acetonitril/Wasser-Gemischen gereinigt. Man erhielt 2,1 g (50 %) der Titelverbindung 2 als hellgelbe, viskose Masse.

¹H-NMR (CDCl₃, δ in ppm): 8,45 (s, 1H); 7,4 (m, 5H); 6,95 (d, 2H); 4,25 (s, 2H); 3,0 (dd, 1H); 2,75 (dd, 1H); 2,45 (s, 3H); 2,05 (m, 1H); 1,25 (m, 1H); 1,1 (m, 1H); 0,8 (t, 3H); 0,7 (d, 3H)

### Beispiel 3: 5-Methyl-6-(2,6-difluor-4-benzylsulfoxylphenyl)-7-(2-methylbutyl)-1,2,4-triazolo[1,5a]pyrimidin (I-5)

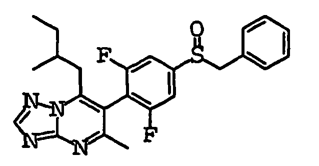

0,9 g (2 mmol) 5-Methyl-6-(2,6-difluor-4-benzylthio-phenyl)-7-(2-methylbutyl)-1,2,4-triazolo[1,5a]pyrimidin (Beispiel 2) in 30 ml Methylenchlorid wurden mit 0,5 g (2,2 mmol) 77 % iger m-Chlorperbenzoesäure versetzt und ca. 2 Stunden bei Raumtemperatur gerührt.

Anschließend gab man verdünnte Natronlauge zur Reaktionsmischung, trennte die Phasen und extrahierte die organische Phase zweimal mit Wasser. Die organische Phase wurde eingeengt und der Rückstand wurde mittels MPLC an Kieselgel RP-18 mit Acetonitril/Wasser-Gemischen gereinigt. Man erhielt 0,7 g (77 %) der Titelverbindung 3 als hellgelbes Öl.

¹H-NMR (CDCl₃, δ in ppm): 8,5 (s, 1H); 7,35 (m, 3H); 7,1 (m, 4H); 4,2 (dd, 2H); 3,05 (dd, 1H); 2,7 (dd, 1H); 2,45 (s, 3H); 2,1 (m, 1H); 1,3 (m, 1H); 1, 1 (m, 1H); 0,8 (t, 3H) ; 0,75 (d, breit, 3H)

### Beispiel 4: 5-Methyl-6-(2,6-difluor-4-benzylsulfonyl-phenyl)-7-(2-methylbutyl)-1,2,4-triazolo[1,5a]pyrimidin (I-6)

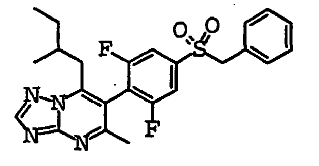

0,4 g (1 mmol) 5-Methyl-6-(2,6-difluor-4-benzylsulfoxyl-phenyl)-7-(2-methylbutyl)-1,2,4-triazolo[1,5a]pyrimidin (Beispiel 3) in 10 ml Methylenchlorid wurden mit 0,3 g (1,3 mmol) 77 % iger m-Chlorperbenzoesäure versetzt und ca. 1 Stunde bei Raumtemperatur gerührt.

Anschließend wurde die Reaktionsmischung mit verdünnter Natronlauge extrahiert und eingeengt und der Rückstand wurde säulenchromatographisch mit Cyclohexan/Essigester-Gemischen gereinigt. Man erhielt 0,25 g (53 %) der Titelverbindung 4 als hellgelbes Öl.

¹H-NMR (CDCl₃, δ inppm): 8,5 (s, 1H) ; 7,35 (m, 5H); 7,2 (d, 2H); 4,45 (s, 2H); 3,0 (dd, 1H); 2,7 (dd, 1H); 2,4 (s, 3H); 2,1 (m, 1H); 1,3 (m, 1H); 1,1 (m, 1H); 0,8 (t, 3H); 0,7 (d; 3H)

**Wirkstoftabelle**

| | | | | | |
|---|---|---|---|---|---|
| 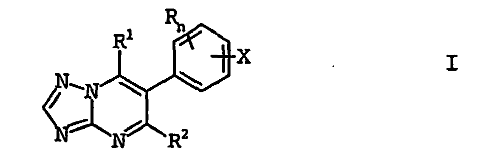 | | | | | |

| Nr. | R¹ | R² | Rₙ | X | Physikalische Daten (Fp [°C], IR [cm⁻¹], ¹H-NMR [ppm] |
|---|---|---|---|---|---|
| I-1 | But-3-en-yl | Methyl | 2,6-F₂ | 4-S-t-C₄H₉ | 100-102 |
| I-2 | But-3-en-yl | Methyl | 2, 6-F₂ | 4-SO₂-t-C₄H₉ | 176-178 |
| I-3 | 2-Methylbutyl | Methyl | 2,6-F₂ | 4-S-Benzyl | 8,45 (s, 1H); 4,25 (s, 2H); 2,45 (s, 3H) |
| I-4 | 2-Methylbutyl | Methyl | 2, 6-F₂ | 4-S-t-C₄H₉ | 95-97 |
| I-5 | 2-Methylbutyl | Methyl | 2,6-F₂ | 4-SO-Benzyl | 8,5 (s, 1H); 4,2 (dd, 2H); 2,45 (s, 3H) |
| I-6 | 2-Methylbutyl | Methyl | 2,6-F₂ | 4-SO₂-Benzyl | 8,5 (s, 1H); 4,45 (s, 2H); 2,4 (s, 3H) |
| I-7 | 2-Methylbutyl | Methyl | 2,6-F₂ | 4-S-CH₃ | 8,45 (s, 1H); 2,6 (s, 3H); 2,45 (s, 3H) |
| I-8 | 2-Methylbutyl | Methyl | 2,6-F₂ | 4-SO-CH₃ | 8,5 (s, 1H); 2,85 (s, 3H); 2,45 (s, 3H) |
| I-9 | 2-Methylbutyl | Methyl | 2,6-F₂ | 4-SO₂-CH₃ | 8,5 (s, 1H); 3,25 (s, 3H); 2,5 (s, 3H) |
| 1-I0 | 2-Methylbutyl | Methyl | 2,6-F₂ | 4-SO₂-n-C₃H₇ | 8,45 (s, 1H); 3,2 (t, 2H); 2,45 (s, 3H) |
| I-11 | 2-Methylbutyl | Methyl | 2,6-F₂ | 4-S-C₂H₅ | 8,45 (s, 1H); 3,1 (q, 2H); 2,45 (s, 3H) |
| I-12 | 2-Methylbutyl | Methyl | 2,6-F₂ | 4-S-n-C₃H₇ | 8,45 (s, 1H); 3,0 (t, 2H); 2,45 (s, 3H) |
| I-13 | 2-Methylbutyl | Methyl | 2,6-F₂ | 4-SO-C₂H₅ | 8,45 (s, 1H); 7,4 (m, 2H); 2,45 (s, 3H) |
| I-14 | 2-Methylbutyl | Methyl | 2,6-F₂ | 4-SO₂-C₂H₅ | 122-124 |
| I-15 | 2-Methylbutyl | Methyl | 2,6-F₂ | 4-SO-n-C₃H₇ | 8,5 (s, 1H); 2,9 (t, 2H); 2,45 (s, 3H) |
| I-16 | 2-Methylbutyl | Methyl | 2-Cl | 4-NH-CO-CH₃ | 8,45 (s, 1H); 2,4 (s, 3H); 2,3 (s, 3H) |

### Beispiele für die Wirkung gegen Schadpilze

Die fungizide Wirkung der Verbindungen der allgemeinen Formel I ließ sich durch die folgenden Versuche zeigen:

Die Wirkstoffe wurden getrennt als Stammlösung formuliert mit 0,25 Gew.-% Wirkstoff in Aceton oder DMSO. Dieser Lösung wurde 1 Gew.-% Emulgator Uniperol® EL (Netzmittel mit Emulgier- und Dispergierwirkung auf der Basis ethoxylierter Alkylphenole) zugesetzt. Die Stammlösungen der Wirkstoffe wurden entsprechend der angegebenen Konzentration mit Wasser verdünnt.

### Anwendungsbeispiele

### Beispiel 1: Wirksamkeit gegen Mehltau an Gurkenblättern verursacht durch Sphaerotheca fuliginea bei protektiver Anwendung

Blätter von in Töpfen gewachsenen Gurkenkeimlingen der Sorte "Chinesische Schlange" wurden im Keimblattstadium mit wässriger Suspension in der unten angegebenen Wirkstoffkonzentration bis zur Tropfnässe besprüht. 20 Stunden nach dem Antrocknen des Spritzbelages wurden die Pflanzen mit einer wässrigen Sporensuspension des Gurkenmehltaus (*Sphaerotheca fuliginea*) inokuliert. Anschließend wurden die Pflanzen im Gewächshaus bei Temperaturen zwischen 20 und 24° C und 60 bis 80 % relativer Luftfeuchtigkeit für 7 Tage kultiviert. Dann wurde das Ausmaß der Mehltauentwicklung visuell in %-Befall der Keimblattfläche ermittelt.

Bei diesem Test zeigten die mit 250 ppm der Verbindungen I-7 und I-8 behandelten Pflanzen einen Befall von ≤ 20 %, während die unbehandelten Kontrollpflanzen zu 90 % von Mehltau befallen waren.

### Beispiel 2: Wirksamkeit gegen die Dürrfleckenkrankheit der Tomate verursacht durch Alternaria solani

Blätter von Topfpflanzen der Sorte "Goldene Prinzessin" wurden mit einer wässriger Suspension in der unten angegebenen Wirkstoffkonzentration bis zur Tropfnässe besprüht. Am folgenden Tag wurden die Blätter mit einer wässrigen Sporenaufschwemmung von *Alternaria solani* in 2 % Biomalzlösung mit einer Dichte von 0.17 x 10⁶ Sporen/ml infiziert. Anschließend wurden die Pflanzen in einer wasserdampf-gesättigten Kammer bei Temperaturen zwischen 20 und 22°C aufgestellt. Nach 5 Tagen hatte sich die Krautfäule auf den unbehandelten, jedoch infizierten Kontrollpflanzen so stark entwickelt, dass der Befall visuell in % ermittelt werden konnte.

Bei diesem Test zeigten die mit 250 ppm der Verbindungen I-4, I-7, I-12 und I-13 behandelten Pflanzen einen Befall von ≤ 30 %, während die unbehandelten (Kontroll)pflanzen durch den Pilzbefall zu 90 % geschädigt waren.

### Beispiel 3: Wirksamkeit gegen Rebenperonospora verursacht durch Plasmopara viticola

Blätter von Topfreben wurden mit wässriger Suspension in der unten angegebenen Wirkstoffkonzentration bis zur Tropfnässe besprüht. Am folgenden Tag wurden die Unterseiten der Blätter mit einer wässrigen Sporangienaufschwemmung von *Plasmopara viticola* inokuliert. Danach wurden die Reben zunächst für 48 Stunden in einer wasserdampfgesättigten Kammer bei 24° C und anschließend für 5 Tage im Gewächshaus bei Temperaturen zwischen 20 und 30° C aufgestellt. Nach dieser Zeit wurden die Pflanzen zur Beschleunigung des Sporangienträgerausbruchs abermals für 16 Stunden in eine feuchte Kammer gestellt. Dann wurde das Ausmaß der Befallsentwicklung auf den Blattunterseiten visuell ermittelt.

Bei diesem Test zeigten die mit 250 ppm der Verbindungen I-4 bis I-7 behandelten Pflanzen einen Befall von ≤ 30 %, während die unbehandelten (Kontroll)pflanzen zu 80 % von Schadpilzen befallen waren.

## Patentansprüche

1. Triazolopyrimidine der Formel I 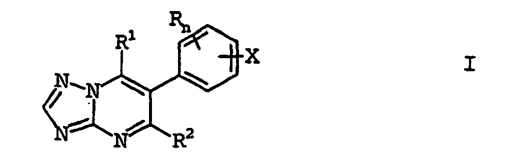 in der der Index und die Substituenten folgende Bedeutung haben:
R¹ C₁-C₁₀-Alkyl, C₂-C₁₀-Alkenyl, C₂-C₁₀-Alkinyl, C₃-C₁₀-Cycloalkyl, C₃-C₁₀-Cycloalkenyl, Phenyl, Naphthyl, oder ein fünf- bis zehngliedriger gesättigter, partiell ungesättigter oder aromatischer Heterocyclus, der über Kohlenstoff mit dem Triazolopyrimidin verbunden ist und ein bis vier Heteroatome aus der Gruppe O, N oder S enthält,
wobei R¹ partiell oder vollständig halogeniert oder durch eine bis vier gleiche oder verschiedene Gruppen R^{a} substituiert sein kann:
R^{a} Halogen, Cyano, Nitro, Hydroxy, C₁-C₆-Alkyl, C₁-C₆-Halogenalkyl, C₁-C₆-Alkylcarbonyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, DiC₁-C₆-alkylamino, C₂-C₆-Alkenyl, C₂-C₆-Alkenyloxy, C₃-C₆-Alkinyloxy, C₃-C₆-Cycloalkyl, Phenyl, Naphthyl, fünf- bis zehngliedriger gesättigter, partiell ungesättigter oder aromatischer Heterocyclus, enthaltend ein bis vier Heteroatome aus der Gruppe O, N oder S, wobei diese aliphatischen, alicyclischen oder aromatischen Gruppen ihrerseits partiell oder vollständig halogeniert sein oder eine bis drei Gruppen R^{b} tragen können:
R^{b} Halogen, Cyano, Nitro, Hydroxy, Mercapto, Amino, Carboxyl, Aminocarbonyl, Aminothiocarbonyl, Alkyl, Alkenyl, Alkinyl, Alkenyloxy, Alkinyloxy, Alkoxy, Alkylthio, Alkylamino, Dialkylamino,
Formyl, Alkylcarbonyl, Alkylsulfonyl, Alkylsulfoxyl, Alkoxycarbonyl, Alkylcarbonyloxy, Alkylaminocarbonyl, Dialkylaminocarbonyl, Alkylaminothiocarbonyl, Dialkylaminothiocarbonyl, wobei die Alkylgruppen in diesen Resten 1 bis 6 Kohlenstoffatome enthalten und die genannten Alkenyl- oder Alkinylgruppen in diesen Resten 2 bis 8 Kohlenstoffatome enthalten und die vorgenannten Gruppen teilweise oder vollständig halogeniert sein können;
und/oder einen bis drei der folgenden Reste:
Cycloalkyl, Cycloalkoxy, Heterocyclyl, Heterocyclyloxy, wobei die cyclischen Systeme 3 bis 10 Ringglieder enthalten; Aryl, Aryloxy, Arylthio, Aryl-C₁-C₆-alkoxy, Aryl-C₁-C₆-alkyl, Hetaryl, Hetaryloxy, Hetarylthio, wobei die Arylreste vorzugsweise 6 bis 10 Ringglieder, die Hetarylreste 5 oder 6 Ringglieder enthalten, wobei die cyclischen Systeme partiell oder vollständig halogeniert oder durch Alkyl- oder Haloalkylgruppen substituiert sein können;
R² C₁-C₄-Alkyl, das durch Halogen, Cyano, Nitro oder C₁-C₂-Alkoxy substituiert sein kann;
n 0 oder eine ganze Zahl von 1 bis 4;
R Halogen, Cyano, C₁-C₆-Alkyl, C₂-C₁₀-Alkenyl, C₂-C₁₀-Alkinyl, C₁-C₆-Halogenalkyl, C₂-C₁₀-Halogenalkenyl, C₁-C₆-Alkoxy, C₂-C₁₀-Alkenyloxy, C₂-C₁₀-Alkinyloxy, C₁-C₆-Halogenalkoxy, C₃-C₆-Cycloalkyl, C₃-C₆-Cycloalkenyl, C₃-C₆-Cycloalkoxy, C₁-C₈-Alkoxycarbonyl, C₂-C₁₀-Alkenyloxycarbonyl, C₂-C₁₀-Alkinyloxycarbonyl, Aminocarbonyl, C₁-C₈-Alkylaminocarbonyl, Di-(C₁-C₈-)alkylaminocarbonyl, C₁-C₈-Alkoximinoalkyl, C₂-C₁₀-Alkenyloximinoalkyl, C₂-C₁₀-Alkinyloximinoalkyl, C₁-C₈-Alkylcarbonyl, C₂-C₁₀-Alkenylcarbonyl, C₂-C₁₀-Alkinylcarbonyl, C₃-C₆-Cycloalkylcarbonyl, oder ein fünf- bis zehngliedriger gesättigter, partiell ungesättigter oder aromatischer Heterocyclus, enthaltend ein bis vier Heteroatome aus der Gruppe O, N oder S;
X SOₘ-R^{x}, NR^{x}R^{y} oder NR^{x}-(C=O)-R^{y};
R^{x}, R^{y}: Wasserstoff, C₁-C₆-Alkyl, C₂-C₁₀-Alkenyl, C₂-C₁₀-Alkinyl, C₃-C₆-Cycloalkyl, C₃-C₆-Cycloalkenyl, wobei die vorstehenden Reste partiell oder vollständig halogeniert sein können oder durch Cyan, C₁-C₄-Alkoximino, C₂-C₄-Alkenyloximino, C₂-C₄-Alkinyloximino oder C₁-C₄-Alkoxy substituiert sein können.
m 0 oder eine ganze Zahl 1 bis 3.

2. Triazolopyrimidine der Formel I' 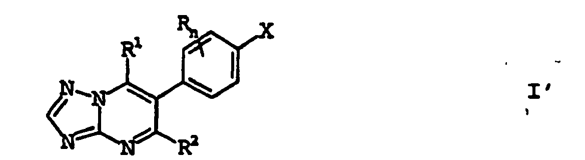 in der der Index und die Substituenten folgende Bedeutung haben:
R¹ C₃-C₈-Alkyl, C₃-C₈-Alkenyl, C₃-C₈-Alkinyl, C₃-C₆-Cycloalkyl, C₅-C₆-Cycloalkenyl; wobei R¹ partiell oder vollständig halogeniert oder durch eine bis vier gleiche oder verschiedene Gruppen R^{a} substituiert sein kann:
R^{a} Halogen, Cyano, C₁-C₆-Alkyl, C₂-C₆-Alkenyl, C₂-C₆-Alkinyl, C₁-C₆-Alkoxy, C₁-C₆-Alkoxycarbonyl, C₁-C₆-Alkoximino, C₂-C₆-Alkenyloximino, C₂-C₆-Alkinyloximino, C₃-C₆-Cycloalkyl, C₅-C₆-Cycloalkenyl, wobei die aliphatischen oder alicyclischen Gruppen ihrerseits partiell oder vollständig halogeniert sein oder eine bis drei Gruppen R^{b} tragen können:
R^{b} Halogen, Cyano, C₁-C₆-Alky , C₁-C₆-Haloalkyl, C₂-C₆-Alkenyl, C₂-C₆-Alkinyl, C₁-C₆-Alkylcarbonyl, C₁-C₆-Haloalkylcarbonyl oder C₁-C₆-Alkoxy:
R² C₁-C₄-Alkyl, das durch Halogen substituiert sein kann;
n eine ganze Zahl von 0 bis 2 :
R Fluor, Chlor, Brom, Cyano, C₁-C₆-Alkyl, C₁-C₆-Alkoxy;
X SO-R^{x}, SO₂-R^{x} oder NR^{x}-(C=O)-R^{y}
R^{x}, R^{y} Wasserstoff, C₁-C₆-Alkyl, C₂-C₆-Alkenyl oder C₃-C₆-Cycloalkyl, wobei die vorstehenden Reste partiell oder vollständig halogeniert sein können.

3. Verfahren zur Herstellung der Verbindungen der Formel I gemäß Ansprüchen 1 und 2 durch Umsetzung von 5-Aminotriazol der **Formel II** 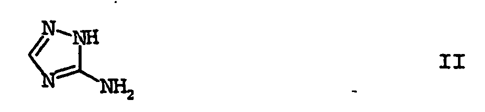 mit Dicarbonylverbindungen der Formel III 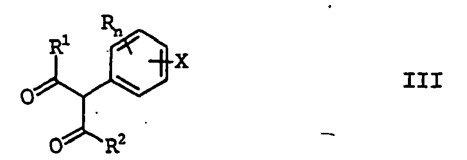 in der die Substituenten R, X, R¹ und R² und der Index n die in Anspruch 1 angegebene Bedeutung haben.

4. Dicarbonylverbindungen der Formel III, die in Anspruch 3 definiert ist.

5. Zur Bekämpfung von Schadpilzen geeignetes Mittel, enthaltend einen festen oder flüssigen Trägerstoff und eine Verbindung der Formel I gemäß Anspruch 1.

6. Verwendung der Verbindungen I gemäß Anspruch 1 zur Herstellung eines zur Bekämpfung von Schadpilzen geeigneten Mittels.

7. Verfahren zur Bekämpfung von Schadpilzen, **dadurch gekennzeichnet, daß** man die Pilze oder die vor Pilzbefall zu schützenden Materialien, Pflanzen, den Boden oder Saatgüter mit einer wirksamen Menge einer Verbindung der Formel I gemäß Anspruch 1 behandelt.

## Claims

1. A triazolopyrimidine of the formula I 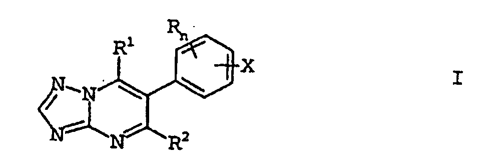 where the index and the substituents are as defined below:
R¹ is C₁-C₁₀-alkyl, C₂-C₁₀-alkenyl, C₂-C₁₀-alkynyl, C₃-C₁₀-cycloalkyl, C₃-C₁₀-cycloalkenyl, phenyl, naphthyl or a five- to ten-membered saturated, partially unsaturated or aromatic heterocycle which is attached via carbon to the triazolopyrimidine and contains one to four heteroatoms from the group consisting of O, N and S,
where R¹ may be partially or fully halogenated or substituted by one to four identical or different groups R^{a}:
R^{a} is halogen, cyano, nitro, hydroxyl, C₁-C₆-alkyl, C₁-C₆-haloalkyl, C₁-C₆-alkylcarbonyl, C₃-C₆-cycloalkyl, C₁-C₆-alkoxy, C₁-C₆-haloalkoxy, C₁-C₆-alkoxycarbonyl, C₁-C₆-alkylthio, C₁-C₆-alkylamino, di-C₁-C₆-alkylamino, C₂-C₆-alkenyl, C₂-C₆-alkenyloxy, C₃-C₆-alkynyloxy, C₃-C₆-cycloalkyl, phenyl, naphthyl, a five- to ten-membered saturated, partially unsaturated or aromatic heterocycle which contains one to four heteroatoms from the group consisting of O, N and S, where these aliphatic, alicyclic or aromatic groups for their part may be partially or fully halogenated or carry one to three groups R^{b}:
R^{b} is halogen, cyano, nitro, hydroxyl, mercapto, amino, carboxyl, aminocarbonyl, aminothiocarbonyl, alkyl, alkenyl, alkynyl, alkenyloxy, alkynyloxy, alkoxy, alkylthio, alkylamino, dialkylamino, formyl, alkylcarbonyl, alkylsulfonyl, alkylsulfoxyl, alkoxycarbonyl, alkylcarbonyloxy, alkylaminocarbonyl, dialkylaminocarbonyl, alkylaminothiocarbonyl, dialkylaminothiocarbonyl, where the alkyl groups in these radicals contain 1 to 6 carbon atoms and the above-mentioned alkenyl or alkynyl groups in these radicals contain 2 to 8 carbon atoms and the above-mentioned groups may be partially or fully halogenated;
and/or one to three of the following radicals:
cycloalkyl, cycloalkoxy, heterocyclyl, heterocyclyloxy, where the cyclic systems contain 3 to 10 ring members; aryl, aryloxy, arylthio, aryl-C₁-C₆-alkoxy, aryl-C₁-C₆-alkyl, hetaryl, hetaryloxy, hetarylthio, where the aryl radicals preferably contain 6 to 10 ring members and the hetaryl radicals 5 or 6 ring members, where the cyclic systems may be partially or fully halogenated or substituted by alkyl or haloalkyl groups;
R² is C₁-C₄-alkyl which may be substituted by halogen, cyano, nitro or C₁-C₂-alkoxy;
n is 0 or an integer from 1 to 4;
R is halogen, cyano, C₁-C₆-alkyl, C₂-C₁₀-alkenyl, C₂-C₁₀-alkynyl, C₁-C₆-haloalkyl, C₂-C₁₀-haloalkenyl, C₁-C₆-alkoxy, C₂-C₁₀-alkenyloxy, C₂-C₁₀-alkynyloxy, C₁-C₆-haloalkoxy, C₃-C₆-cycloalkyl, C₃-C₆-cycloalkenyl, C₃-C₆-cycloalkoxy, C₁-C₈-alkoxycarbonyl, C₂-C₁₀-alkenyloxycarbonyl, C₂-C₁₀-alkynyloxycarbonyl, aminocarbonyl, C₁-C₈-alkylaminocarbonyl, di-(C₁-C₈-)alkylaminocarbonyl, C₁-C₈-alkoximinoalkyl, C₂-C₁₀-alkenyloximinoalkyl, C₂-C₁₀-alkynyloximinoalkyl, C₁-C₈-alkylcarbonyl, C₂-C₁₀-alkenylcarbonyl, C₂-C₁₀-alkynylcarbonyl, C₃-C₆-cycloalkylcarbonyl, or a five- to ten-membered saturated, partially unsaturated or aromatic heterocycle which contains one to four heteroatoms from the group consisting of O, N and S;
X is SOₘ-R^{x}, NR^{x}R^{y} or NR^{x}-(C=O)-R^{y}
R^{x}, R^{y} are: hydrogen, C₁-C₆-alkyl, C₂-C₁₀-alkenyl, C₂-C₁₀-alkynyl, C₃-C₆-cycloalkyl, C₃-C₆-cydoalkenyl, where the above radicals may be partially or fully halogenated or substituted by cyano, C₁-C₄-alkoximino, C₂-C₄-alkenyloximino, C₂-C₄-alkynyloximino or C₁-C₄-alkoxy;
m is 0 or an integer from 1 to 3.

2. A triazolopyrimidine of the formula I' 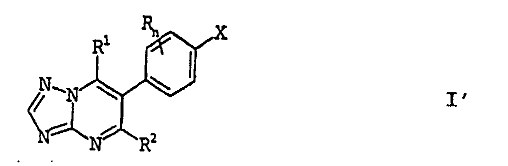 where the index and the substituents are as defined below:
R¹ is C₃-C₈-alkyl, C₃-C₈-alkenyl, C₃-C₈-alkynyl, C₃-C₆-cycloalkyl, C₅-C₆-cycloalkenyl; where R¹ may be partially or fully halogenated or substituted by one to four identical or different groups R^{a}:
R^{a} is halogen, cyano, C₁-C₆-alkyl, C₂-C₆-alkenyl, C₂-C₆-alkynyl, C₁-C₆-alkoxy, C₁-C₆-alkoxycarbonyl, C₁-C₆-alkoximino, C₂-C₆-alkenyloximino, C₂-C₆-alkynyloximino, C₃-C₆-cycloalkyl, C₅-C₆-cycloalkenyl, where the aliphatic or alicyclic groups for their part may be partially or fully halogenated or carry one to three groups R^{b}:
R^{b} is halogen, cyano, C₁-C₆-alkyl, C₁-C₆-haloalkyl, C₂-C₆-alkenyl, C₂-C₆-alkynyl, C₁-C₆-alkylcarbonyl, C₁-C₆-haloalkylcarbonyl or C₁-C₆-alkoxy;
R² is C₁-C₄-alkyl which may be substituted by halogen;
n is an integer from 0 to 2;
R is fluorine, chlorine, bromine, cyano, C₁-C₆-alkyl, C₁-C₆-alkoxy;
X is SO-R^{x}, SO₂-R^{x} or NR^{x}-(C=O)-R^{y};
R^{x}, R^{y} are: hydrogen, C₁-C₆-alkyl, C₂-C₆-alkenyl or C₃-C₆-cycloalkyl, where the above radicals may be partially or fully halogenated.

3. A process for preparing compounds of the formula I as claimed in claim 1 or 2 which comprises reacting 5-aminotriazole of the formula II 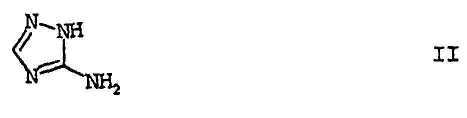 with dicarbonyl compounds of the formula III 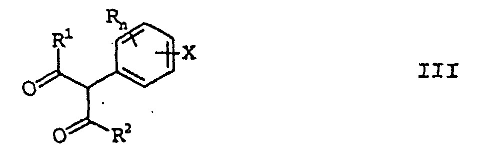 where the substituents R, X, R¹ and R² and the index n are as defined in claim 1.

4. A dicarbonyl compound of the formula III, which is defined in claim 3.

5. A composition suitable for controlling harmful fungi, comprising a solid or liquid carrier and a compound of the formula I as claimed in claim 1.

6. The use of the compounds I as claimed in claim 1 for preparing a composition suitable for controlling harmful fungi.

7. A method for controlling harmful fungi, which comprises treating the fungi or the materials, plants, the soil or seeds to be protected against fungal attack with an effective amount of a compound of the formula I as claimed in claim 1.

## Revendications

1. Triazolopyrimidines de formule I : 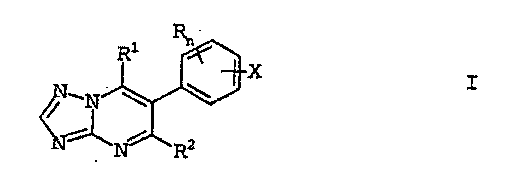 dans laquelle l'indice et les substituants ont la signification suivante :
R¹ représente un groupe alkyle en C₁-C₁₀, alcényle en C₂-C₁₀, alcynyle en C₂-C₁₀, cycloalkyle en C₃-C₁₀, cycloalcényle en C₃-C₁₀, phényle, naphtyle ou un hétérocycle saturé, partiellement insaturé ou aromatique pentavalent à décavalent qui est relié par l'intermédiaire d'un carbone à la triazolopyrimidine et contient un à quatre hétéroatomes du groupe O, N ou S,
R¹ pouvant être partiellement ou totalement halogéné ou être substitué par un à quatre groupes R^{a} identiques ou différents :
R^{a} représente de l'halogène ou un groupe cyano, nitro, hydroxy, alkyle en C₁-C₆, halogénoalkyle en C₁-C₆, alkylcarbonyle en C₁-C₆, cycloalkyle en C₃-C₆, alcoxy en C₁-C₆, halogénoalcoxy en C₁-C₆, alcoxycarbonyle en C₁-C₆, alkylthio en C₁-C₆, alkylamino en C₁-C₆, di-C₁-C₆-alkylamino, alcényle en C₂-C₆, alcényloxy en C₂-C₆, alcynyloxy en C₃-C₆, cycloalkyle en C₃-C₆, phényle, naphtyle, ou un hétérocycle saturé, partiellement insaturé ou aromatique pentavalent à décavalent, contenant un à quatre hétéroatomes du groupe de O, N ou S, ces groupes aliphatiques, alicycliques ou aromatiques pouvant à leur tour être partiellement ou totalement halogénés ou porter un à trois groupes R^{b} ;
R^{b} représente de l'halogène ou un groupe cyano, nitro, hydroxy, mercapto, amino, carboxyle, aminocarbonyle, aminothiocarbonyle, alkyle, alcényle, alcynyle, alcényloxy, alcynyloxy, alcoxy, alkylthio, alkylamino, dialkylamino, formyle, alkylcarbonyle, alkylsulfonyle, alkylsulfoxyle, alcoxycarbonyle, alkylcarbonyloxy, alkylaminocarbonyle, dialkylaminocarbonyle, alkylaminothiocarbonyle, dialkylaminothiocarbonyle, les groupes alkyle dans ces radicaux contenant 1 à 6 atomes de carbone et les groupes alcényle ou alcynyle cités dans ces radicaux contenant 2 à 8 atomes de carbone et lesdits groupes pouvant être partiellement ou totalement halogénés ;
et/ou un à trois des radicaux suivants :
cycloalkyle, cycloalcoxy, hétérocyclyle, hétérocyclyloxy, où les systèmes cycliques contiennent 3 à 10 termes cycliques ; aryle, aryloxy, arylthio, aryl-C₁-C₆-alcoxy, aryl-C₁-C₆-alkyle, hétaryle, hétaryloxy,
hétarylthio, où les radicaux aryle contiennent de préférence 6 à 10 termes cycliques, les radicaux hétaryle 5 ou 6 termes cycliques, les systèmes cycliques pouvant être partiellement ou totalement halogénés ou substitués par des groupes alkyle ou halogénoalkyle ;
R² représente un groupe alkyle en C₁-C₄, pouvant être substitué par de l'halogène ou un groupe cyano, nitro, ou alcoxy en C₁-C₂,
n désigne 0 ou un nombre entier de 1 à 4 ;
R représente de l'halogène ou un groupe cyano, alkyle en C₁-C₆, alcényle en C₂-C₁₀, alcynyle en C₂-C₁₀, halogénoalkyle en C₁-C₆, halogénoalcényle en C₂-C₁₀, alcoxy en C₁-C₆, alcényloxy en C₂-C₁₀, alcynyloxy en C₂-C₁₀, halogénoalcoxy en C₁-C₆, cycloalkyle en C₃-C₆, cycloalcényle en C₃-C₆, cycloalcoxy en C₃-C₆, alcoxycarbonyle en C₁-C₈, alcényloxycarbonyle en C₂-C₁₀, alcynyloxycarbonyle en C₂-C₁₀, aminocarbonyle, alkylaminocarbonyle en C₁-C₈, di-(C₁-C₈-)alkylaminocarbonyle, alcoximinoalkyle en C₁-C₈, alcényloximinoalkyle en C₂-C₁₀, alcynyloximinoalkyle en C₂-C₁₀, alkylcarbonyle en C₁-C₈, alcénylcarbonyle en C₂-C₁₀, alcynylcarbonyle en C₂-C₁₀, cycloalkylcarbonyle en C₃-C₆, ou un hétérocycle saturé, partiellement insaturé ou aromatique pentavalent à décavalent, contenant 1 à 4 hétéroatomes du groupe de O, N ou S ;
X SOₘ-R^{x}, NR^{x}R^{y} ou NR^{x}-(C=O)-R^{y} ;
R^{x}, R^{y} : désignent l'hydrogène, un groupe alkyle en C₁-C₆, alcényle en C₂-C₁₀, alcynyle en C₂-C₁₀, cycloalkyle en C₃-C₆, cycloalcényle en C₃-C₆, les radicaux précités pouvant être partiellement ou totalement halogénés ou substitués par un radical cyano, alcoximino en C₁-C₄, alcényloximino en C₂-C₄, alcynyloximino en C₂-C₄ ou alcoxy en C₁-C₄ ;
m désigne 0 ou un nombre entier de 1 à 3.

2. Triazolopyrimidines de la formule I' 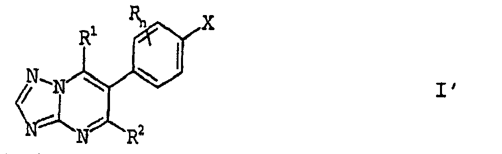 dans laquelle l'indice et les substituants ont la signification suivante :
R¹ représente un groupe alkyle en C₃-C₈, alcényle en C₃-C₈, alcynyle en C₃-C₈, cycloalkyle en C₃-C₆, cycloalcényle en C₅-C₆, R¹ pouvant être partiellement ou totalement halogéné ou être substitué par un à quatre groupes R^{a} identiques ou différents ;
R^{a} représente de l'halogène ou un groupe cyano, alkyle en C₁-C₆, alcényle en C₂-C₆, alcynyle en C₂-C₆, alcoxy en C₁-C₆, alcoxycarbonyle en C₁-C₆, alcoximino en C₁-C₆, alcényloximino en C₂-C₆, alcynyloximino en C₂-C₆, cycloalkyle en C₃-C₆, cycloalcényle en C₅-C₆, ces groupes aliphatiques ou alicycliques pouvant à leur tour être partiellement ou totalement halogénés ou porter un à trois groupes R^{b} :
R^{b} représente de l'halogène, un groupe cyano, alkyle en C₁-C₆, halogénoalkyle en C₁-C₆, alcényle en C₂-C₆, alcynyle en C₂-C₆, alkylcarbonyle en C₁-C₆, halogénoalkylcarbonyle en C₁-C₆ ou alcoxy en C₁-C₆,
R² représente un groupe alkyle en C₁-C₄, qui peut être substitué par de l'halogène ;
n désigne un nombre entier de 0 à 2 ;
R désigne le fluor, le chlore, le brome, un groupe cyano, alkyle en C₁-C₆, alcoxy en C₁-C₆ ;
X désigne SO-R^{x}, SO₂-R^{x} ou NR^{x}-(C=O)-R^{y} ;
R^{x}, R^{y} désignent l'hydrogène, un groupe alkyle en C₁-C₆, alcényle en C₂-C₆ ou cycloalkyle en C₃-C₆, les radicaux précédents pouvant être partiellement ou totalement halogénés.

3. Procédé de préparation des composés de formule I conformément aux revendications 1 et 2 par mise en réaction de 5-aminotriazole de formule II 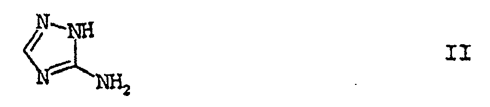 avec des composés dicarbonyliques de formule III 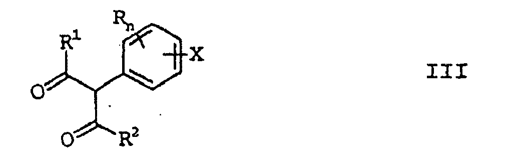 dans laquelle les substituants R, X, R¹ et R² et l'indice n ont la signification indiquée dans la revendication 1.

4. Composés dicarbonyliques de formule III qui sont définis dans la revendication 3.

5. Produit approprié pour lutter contre les champignons nuisibles, contenant un support solide ou liquide et un composé de formule I suivant la revendication 1.

6. Utilisation des composés I suivant la revendication 1, pour la préparation d'un produit approprié pour lutter contre les champignons nuisibles.

7. Procédé de lutte contre les champignons nuisibles, **caractérisé en ce qu'**on traite les champignons ou les matières, plantes, sols ou semences à protéger d'une infestation par ces champignons avec une quantité efficace d'un composé de formule I suivant la revendication 1.
